# EUROPEAN PATENT SPECIFICATION

(11) **EP 2 976 996 B1**
(45) Date of publication and mention of the grant of the patent: **29.05.2019**
(21) Application number: 15178547.4
(22) Date of filing: 27.07.2015
(51) Int. Cl.: G06F 19/00, H04W 68/00, A61B 5/00, H04W 88/04

(54) **NURSE CALL SYSTEM HAVING LOCAL OPERATION MODE**
PFLEGERUFSYSTEM MIT LOKALEM BETRIEBSMODUS
SYSTÈME D'APPEL D'INFIRMIÈRE À MODE DE FONCTIONNEMENT LOCAL

(30) Priority: 25.07.2014 EP 14178642
(43) Date of publication of application: 27.01.2016
(73) Proprietor: Televic Healthcare NV, 8870 Izegem (BE)
(72) Inventor: CROMBEZ, Pieter, 8820 Torhout (BE); GESQUIERE, John, 8970 Poperinge (BE); VERHAEGHE, Geert, 8680 Koekelare (BE); DESMET, Ludwig, 8770 Ingelmunster (BE)
(74) Representative: Beck, Michaël Andries T.

(56) References cited:
- EP-A2- 1 400 941
- WO-A1-2010/150031
- US-A1- 2005 135 236
- US-A1- 2009 003 216
- US-A1- 2009 212 925
- US-A1- 2012 225 655

## Description

### Field of the Invention

The present invention relates to the field of communication systems, more in particular to the field of nurse call systems as used in hospitals and similar facilities.

### Background

Nurse call systems are communication networks designed to relay, *inter alia,* patient localization information and distress calls to a central system, which in turn pages personnel to respond to the situation or the call.

As nurse call systems should allow people to quickly call assistance in emergency situations, security against system failure and the ability to operate flawlessly at all times are of utmost importance. The VDE standard (DIN VDE 0834) takes these considerations into account. It prescribes that the call system be completely independent of elements that are not part of the system. Data transfer with other systems is only allowed when using the system's own interfaces.

Accordingly, for safety reasons, nurse call systems are required to be able to fall back from their normal "networked operation" mode to a "local operation" mode in the event of a loss of network connectivity. In such a case, the hardware will provide highly noticeable visual clues indicating that "local operation" mode is active, and, if necessary, use audio-visual signaling to indicate the presence of a call or the need for a personnel intervention.

It is a disadvantage of the known nurse-call systems that one or more localized network defects can seriously impair the system's ability to continue working in "networked operation" mode.

It is therefore an object of embodiments of the present invention to at least partially overcome said disadvantage.

The application US 2012/0225655 discloses an apparatus and a method for providing a relay service in a Base Station of a communication system.

The method includes detecting a communication state with a backhaul of the communication system, transmitting backhaul error occurrence information to at least one Mobile Station (MS) located in a service coverage area when communication with the backhaul is interrupted, selecting a neighbor BS from among one or more neighbor BSs as a target BS, and providing a relay service by connecting with the target BS.

The application US 2009/0212925 discloses a user station configurable for use in a healthcare communication system, such as a nurse call system.

The application US 2009 0003216 describes a method of transmitting data across a wireless mesh network which uses network coding at each of the intermediate nodes between the source node and the destination node.

Each intermediate node also controls the rate at which it broadcasts packets based on link congestion and the backlog of packets at each of the possible next-hop nodes for the data flow.

### Summary of the Invention

The invention is defined by the system of independent claim 1.

### Brief Description of the Figures

These and other features and advantages of embodiments of the present invention will now be described in more detail with reference to the accompanying drawings, in which:
Figure 1 schematically illustrates a network in which embodiments of the present invention may be used;
Figure 2 schematically illustrates a network in which embodiments of the present invention may be used, including additional details;
Figure 3 illustrates a typical configuration in a building, such as a hospital building, in which embodiments of the present invention may be used;
Figure 4 illustrates an embodiment of the invention, in which a beacon apparatus communicates with other beacon apparatus of a beacon group via consecutive wireless links; and
Figures 5-10 provide flow chart of certain aspects of the operation of beacon apparatus according to embodiments of the present invention.

### Description of embodiments

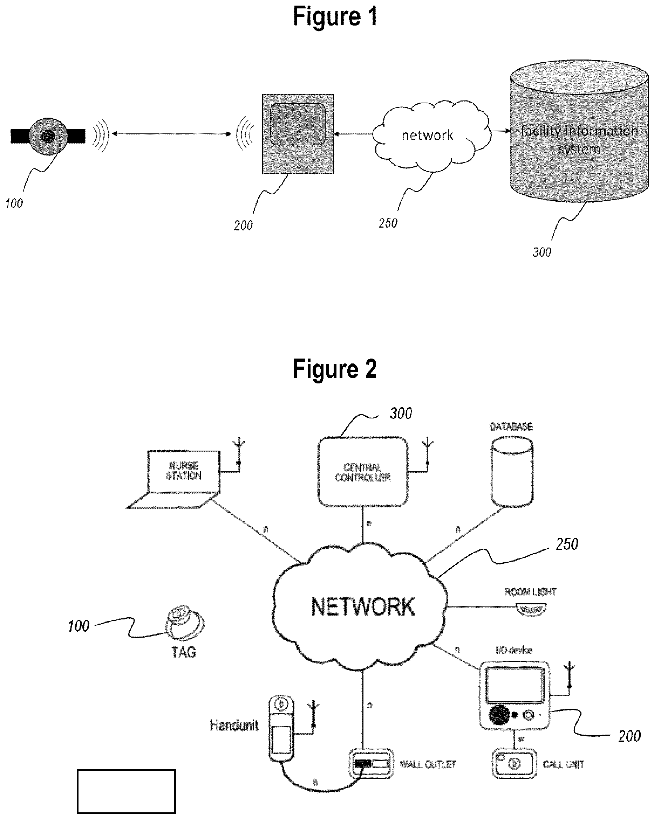

Figure 1 schematically illustrates a nurse call localization system in which embodiments of the present invention may be used.

A specific application of the location system described herein is a wireless nurse call system for use in hospitals and other institutions where patients may move about, possibly without being fully conscious of their own exact location. Where appropriate, the invention will be described with reference to such a nurse call system, without intent to limit the scope of the invention to such applications. In the context of a wireless nurse call system, the development of efficient hardware and efficient communication protocols is an important goal, with a view to reducing (battery) power consumption, obtaining a small form factor, and keeping the total cost as low as possible.

In the preferred localization system, beacons **200** are provided at fixed locations throughout an area in which the location of mobile objects or persons is to be monitored. The beacons may generally be mounted to walls, doors, pillars, and the like. They may have a basic user interface comprising a display and one or more keys.

The beacons emit a signal including an identification element. The mobile objects or persons to be monitored are provided with identification tags (hereinafter also referred to as "tags") **100,** which comprise a receiver for the signals emitted by the beacons **200.**

The tag **100** further comprises communication means to relay the decoded beacon identification element, along with its own identity, to the central monitoring system, in the form of a localization message. The communication means may include a radio frequency (RF) transmitter adapted to wirelessly communicate the information to a beacon (the same beacon whose identification element was received and/or another beacon within radio range), which is in turn connected to a wired network **250** that allows it to communicate with a centralized management system or server **300.**

The beacon apparatus **200** is adapted to receive the identification messages from the tag **100** via a radio frequency transceiver. The nurse call system as considered in this invention can be expanded to cater different needs such as intercom, domotics control, access control, asset tracking and more, and the beacon apparatus **200** will be equipped accordingly.

This first beacon apparatus **200** is connected to a wired backbone network **250,** which connects the beacon apparatus **200** to a server **300.** The wired backbone network **250** is typically a Local Area Network (LAN), which may comprise segments that comply with various LAN standards. At the physical and data link layer, the network advantageously comprises a bridged LAN including segments that operate according to IEEE Std 802.3 (commonly referred to as "Ethernet", which includes specifications for communication over twisted pair cabling, coaxial cabling, and optical fiber). At the network layer, communication may take place by means of the Internet Protocol (IP). The skilled person will appreciate that various other standardized and proprietary networking protocols may be used instead. The use of standardized network equipment simplifies service and allows easy exchange or expansion of devices in rooms. Additional nurse call functions such as VoIP-telephony can easily be implemented.

While traditional Ethernet-based networks are known to be nondeterministic, they can be used for mission critical applications. Ethernet can be used if sufficient bandwidth is provided in view of expected peak loads, and if adequate backup communication means are present. However, industry practices require that all communication infrastructure is provided by the vendor of the nurse call system, in order to keep the responsibility for the correct functioning of the system unambiguous. Thus, if third-party network equipment is used, it is highly desirable to also have a back-up path made up entirely of equipment provided by the vendor of the nurse call system. The present invention is based *inter alia* on the insight of the inventors that a nurse call system may be designed in such a way that the radio-frequency transceivers that are already present in the beacon apparatus may assume the role of back-up communication means, to ensure an enhanced form of local operation when connection to the server is lost or partially lost.

Figure 2 schematically illustrates a nurse call system with additional components. A hand call and/or handset may be connected to a wall outlet via a flexible network cable **h.** Other network connections (not necessarily flexible) are designated in the drawing by the symbol **n.** The outlet connects the handset cable to the network. In the topology of Figure 2, the call unit is in connection with the I/O device via a passive link **w.** The I/O device itself is connected to the network through a network cable **n.** An I/O device may have display capabilities (e.g., an LCD or a touchscreen) and act as a terminal.

Other components of the nurse call system that are shown in Figure 2 include a nurse station, a central controller and a database. These components are also linked with each other through network connections **n.** The database allows storage of information concerning any errors detected in the nurse call system, which can be useful for later analysis. Immediately required interventions are typically displayed at the nurse station. Additionally or alternatively, these messages may be forwarded by the central controller to third-party systems like digital cellular phones, DECT handsets, pagers, and the like.

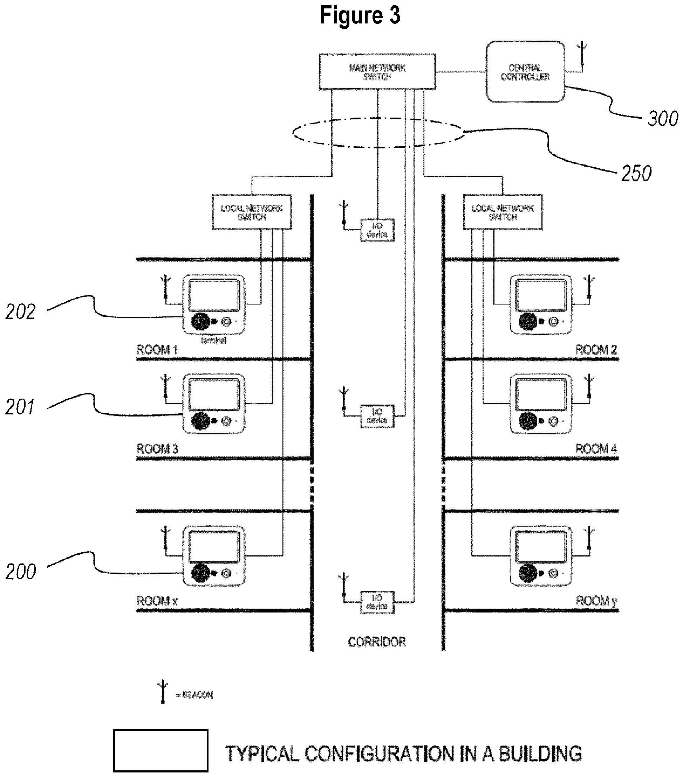

A vital part in a nurse call system is formed by the devices the patients and nursing staff have access to, hereafter called user devices. The principal user devices are considered to be the I/O device **200,** the hand unit, the central controller **300** and the nurse station. User devices are typically found in a patient room. This room is usually part of a larger building as is illustrated in Figure 3. Depending on the needs and wishes of the users, extra I/O devices may be located in this building outside the patient rooms. The number and layout of the rooms and their beacon apparatus as illustrated in the Figures is purely exemplary, and not intended to limit the scope of the invention.

The user devices (and, optionally, the central controller) include beacons, which can send and receive wireless messages. Devices including a beacon are referred to herein as "beacon apparatus" **200-204.**

In embodiments of the present invention, information about calls made from the user devices is stored in a "call list", which is distributed and synchronized between the beacon apparatus of a certain beacon group when none of the members of this beacon group has access to the server. Members of personnel can then access up-to-date information about outstanding calls by checking into any of the user devices belonging to the beacon group.

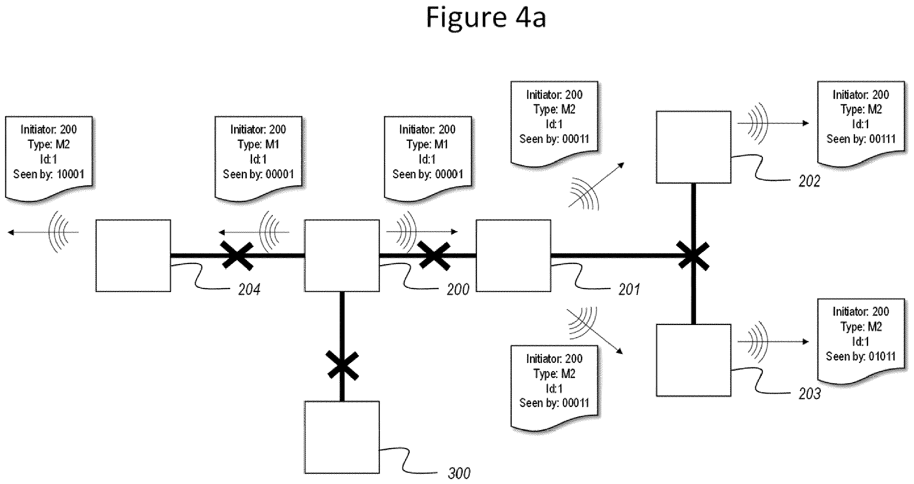

The distribution of messages according to embodiments of the present invention will now be described in more detail with reference to Figures 4a-c.

Each beacon apparatus **200-204** keeps track of its own state (nurse/doctor call placed or not). In addition, each beacon apparatus **200-204** is adapted to store a list of outstanding calls at other beacon apparatus of the beacon group; this list will be referred to as the call list.

In the scenario illustrated in Figures 4a-c, a beacon apparatus **200** communicates with other beacon apparatus **201-204** of a beacon group via consecutive wireless links. This may be the case when all beacon apparatus **200-204** of the beacon group are connected to the server **300** via a common switch or router (not illustrated), and this switch or router goes out of service.

It should be noted that the invention also works in situations where some or all wired links between the beacon apparatus are still available (not illustrated). This could be the case when the server **300** is out of service, but the network infrastructure remains operational. In such cases, the broadcasts required by embodiments of the invention could take place via the wired and the wireless medium at the same time or consecutively. Without loss of generality, the invention is further described with respect to an all-wireless situation.

The distribution of nurse call information may be triggered by the detection at any beacon apparatus of a loss of connectivity to the server **300.** As it is possible that several beacon apparatus simultaneously detect a loss connectivity, it is preferable to implement a random back-off delay in the beacon apparatus before starting the transmission of call list distribution messages, to avoid message collisions.

The distribution of updated nurse call information may also be triggered by the receipt of a call or a call cancellation at the beacon in question.

The distribution of nurse call information may also be triggered by the occurrence of a timed regime change (e.g., switch from day watch to night watch), in which case individual beacon apparatus may be reassigned to different beacon groups. If the distribution is triggered by a regime change, the beacon apparatus should be able to determine in a deterministic way who has the initiative to start the distribution process (e.g., as a function of uniquely assigned beacon identifiers).

Without loss of generality, the following description is based on the assumption that the distribution is first triggered at beacon apparatus **200,** as shown in Figure 4a. Beacon apparatus **200** will attempt to broadcast its version of the call list to the other members of the beacon group via its radio-frequency transceiver. This initial message is illustrated with an exemplary type field value of "M1". It may be advantageous to detect, later on, whether subsequent messages belong to a distribution flow set up by this particular beacon apparatus **200.** While this detection may be performed on the basis of a message identifier, it is also possible to include an identifier of the initiating beacon apparatus in the M1 message and the subsequent messages. This element is shown as the "Initiator" field in the figures.

In particular, the message broadcast by beacon apparatus **200** will reach neighboring beacon apparatus **201, 204** (these beacon apparatus are referred to as the "second beacon apparatus" in the description of claimed method). When a neighboring beacon apparatus **201, 204** receives the broadcast, it will verify whether the broadcast pertains to its beacon group and, if so, it will relay the message with the call list via its own radio-frequency transceiver to the next neighbors in range **202, 203.** Alternatively, beacon apparatus may be configured to relay call list messages regardless of whether they are from a beacon apparatus in the same beacon group, to improve the chances of actually reaching all members of the beacon group; in that case, it is preferred to limit the total number of permitted relay hops (e.g., by including a "time-to-live" field in the message, which gets decremented on every relay hop), to avoid flooding the entire facility's network with messages. The relayed messages are illustrated with an exemplary type field value of "M2".

This "ripple process" continues until the call list has reached all members of the beacon group (the beacon apparatus that relay M2 messages are referred to as the "third beacon apparatus" in the description of claimed method). Optionally, a beacon apparatus that processes the call list may update the call list by adding locally stored call list information, insofar as it is able to ascertain that its locally stored information is up to date (for instance, on the basis of optional timestamps included with the call and clearance events stored in the relayed call list), so as to ensure that the most complete and up-to-date version of the call list is relayed.

In order to avoid that the ripple process might lead to infinite relay loops, each beacon apparatus that passes on the message preferably keeps track of the messages it has already relayed - all call list messages preferably carry a unique identification number for this purpose (shown as the "Id" field in Figures 4a-4c) .

In the interest of determining whether all beacon apparatus of the group have received the call list update, the call list messages preferably carry a "Seen by" field, which is updated upon every relaying step. The "Seen by" field may take the form of a bit map, in which each member of the beacon group has a pre-assigned bit position, which it sets when it processes the message (shown as the "Seen by" field in Figures 4a-4c).

Whenever one of the beacons of the beacon group transmits a call list message via its radio-frequency transceiver, it will be able to detect via that same radio-frequency transceiver whether any neighboring beacon apparatus passes the message on. If the beacon apparatus that initiates the distribution detects that its message is not being relayed, it may conclude that it is isolated, and may switch to fully local operation, which may include emitting visual and auditory signals to attract attention from personnel.

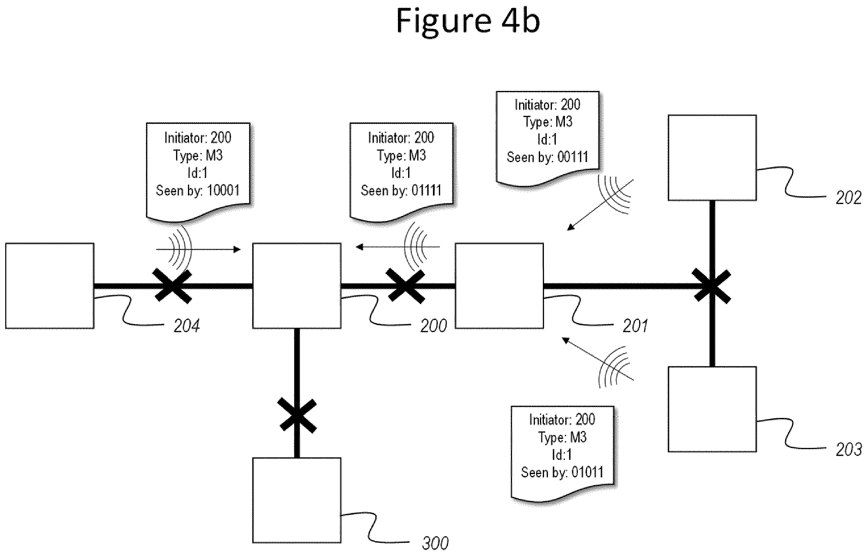

If a downstream beacon apparatus (i.e., a "third beacon apparatus") detects that its message is not being relayed, it may conclude that it is the last node of a sector of the network. In the illustrated example, this is the case for beacon apparatus **202, 203,** and **204.** In that case, the beacon apparatus starts a reverse ripple communication, with a view to distributing the "Seen by" information back to the originating beacon. This reverse ripple is illustrated in Figure 4b. Preferably, the end point messages of the reverse ripple contain a reference to the unique identification of the original call list message (shown as the "Id" field in Figure 4b, which is identical to that of Figure 4a), and a message type indication that distinguishes it from the latter (shown in Figure 4b as a type field with an exemplary value of "M3", which is different from that of Figure 4a). In embodiments where beacon apparatus are allowed to add records to the call list in the downstream ripple, the updated call list should also be included in the upstream ripple messages (not illustrated).

The reverse ripple consists of the original end point messages, and corresponding M3 messages relayed by the beacon apparatus upstream from the end points (these beacon apparatus are referred to as the "fourth beacon apparatus"). In the illustrated example, this is the case for beacon apparatus **201** and **204.** Optionally, there may be further relaying steps by beacon apparatus that received the aforementioned corresponding M3 messages (these beacon apparatus are referred to as the "fifth beacon apparatus").

It is clear from the above that the second and third beacon apparatus on the one hand, and the fourth and fifth beacon apparatus on the other hand, partly refer to the same beacon apparatus.

When reverse ripple messages from different sector end points converge in a common intermediate beacon apparatus, the beacon apparatus may merge these messages (for instance by applying an OR operation to the respective bitmaps), and relay the result. In the illustrated example, this is the case for beacon apparatus **201,** which receives reports from beacon apparatus **202** and **203.** In embodiments where beacon apparatus are allowed to add records to the call list in the downstream ripple, the updated call lists may be merged in the same way (not illustrated).

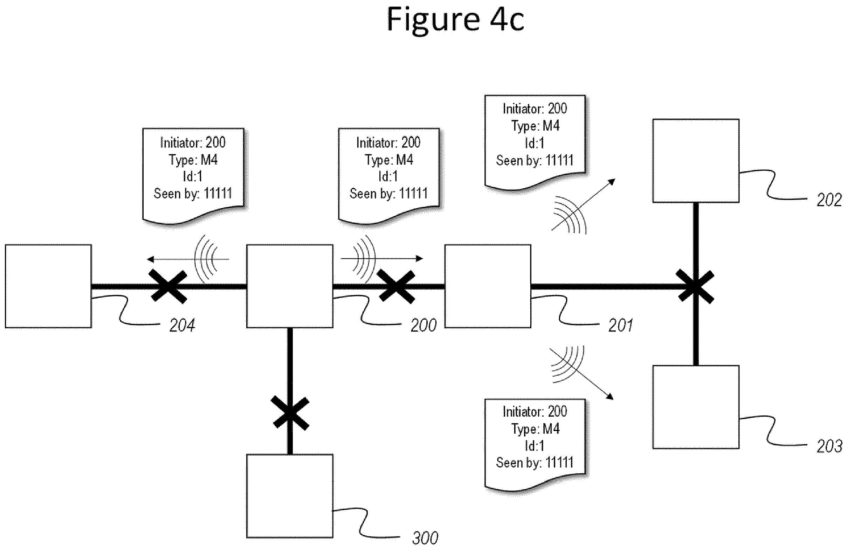

When the reverse ripple message(s) reach the originating beacon, a third ripple is started to distribute the combined "Seen by" field, and optionally, the updated call list, to all members of the beacon group. This third ripple is illustrated in Figure 4c. Preferably, the third ripple message contains a reference to the unique identification of the original call list message (shown as the "Id" field in Figure 4c, which is identical to that of Figures 4a and 4b), and a message type indication that distinguishes it from the latter (shown in Figure 4c as a type field value of "M4", which is different from that of Figures 4a and 4b). Only after this third ripple has taken place, all beacon apparatus will be able to (1) present an up-to-date call list to any member of personnel checking in at that beacon apparatus, and (2) confirm that the list is indeed complete, by indicating that all beacon group members have processed the call list message. If any beacon group member is missing from the "seen-by" list, the checked beacon will also be able to give an indication of that fact, which will allow personnel to treat the missing beacon apparatus as isolated.

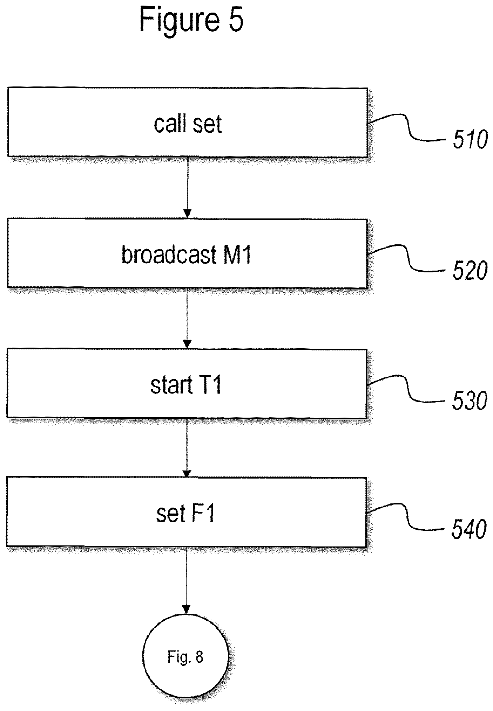

Certain aspects of the method according to embodiments of the present invention will now be explained in more detail with reference to the flow charts provided in Figures 5-9.

In Figure 5, a call list update distribution is triggered by the occurrence of a new call **510.** The beacon apparatus at which the call is set, initiates the downstream ripple by broadcasting **520** a call list update message of the first type (M1). Upon sending this message, it sets a time-out T1, sets flag F1, and listens for relaying of its message by neighboring beacon apparatus. If no relaying is detected by the expiry of T1, the originating beacon apparatus decides that it is isolated.

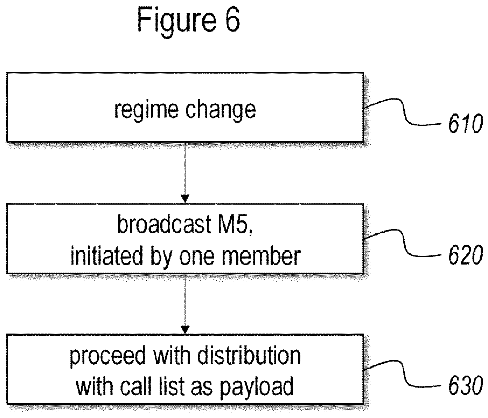

In Figure 6, a regime change **610** takes place, for instance a timed regime change which occurs without human intervention. In that case, a pre-designated member of the beacon group initiates **620** the distribution of the then-current call list. The distribution happens through the ripple message protocol **630** described above, with a message such as or similar to the M1 message described above, using a "Seen by" field whose size reflects the new composition of the beacon group, whereby it may again be useful to allow downstream beacon apparatus to modify the call list under relay, to facilitate the merging of call lists of previously separate beacon groups.

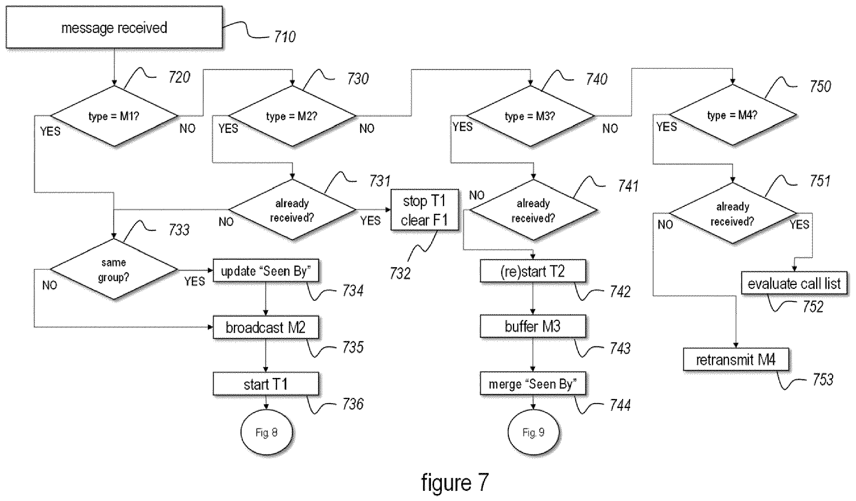

Figure 7 schematically illustrates the processing carried out by an exemplary beacon apparatus according to an embodiment of the present invention, upon receipt **710** of a call list distribution message. The processing depends on the received message type. The selection of the appropriate processing is schematically illustrated as a cascade of type field evaluations **720, 730, 740, 750,** but the skilled person will understand that this evaluation does not have to be implemented in a sequential manner.

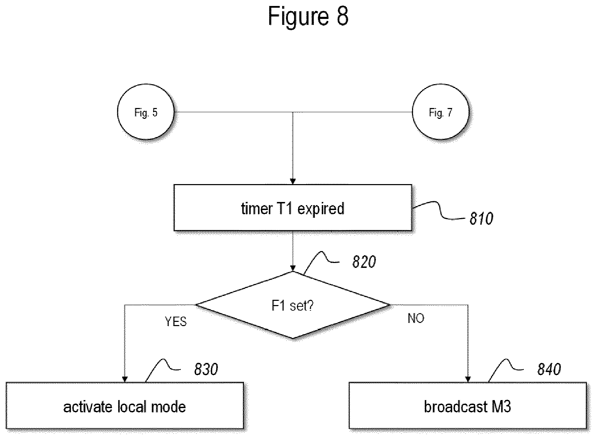

If the message type is found to be "M1", i.e. an initial call list distribution message as transmitted by the beacon apparatus that has taken the initiative of starting a distribution, the receiving beacon apparatus will preferably assess **733** whether the message pertains to the same beacon group as the one it belongs to. If this is the case, it updates **734** the "Seen by" field, which may take the form of a bitmap as explained above. The receiving beacon apparatus then proceeds by retransmitting **735** the call list information, with the optionally updated "Seen by" field; this new message is now designated as an "M2" message. It also starts **736** a timer T1 (see also Figure 8).

If the message type is found to be "M2", i.e. a downstream call list distribution message that has been relayed at least once, the receiving beacon apparatus will assess **731,** preferably on the basis of a unique identifier of the message or the specific distribution flow, whether the message has been received before. If this is the case, the receiving beacon apparatus stops **732** any timer T1 that may be running with respect to that message (see below) and clears flag F1 (indicating that the beacon apparatus is not isolated). If it is not the case, the receiving beacon apparatus will preferably assess **733** whether the message pertains to the same beacon group as the one it belongs to, and proceed as described above for messages of the "M1" type.

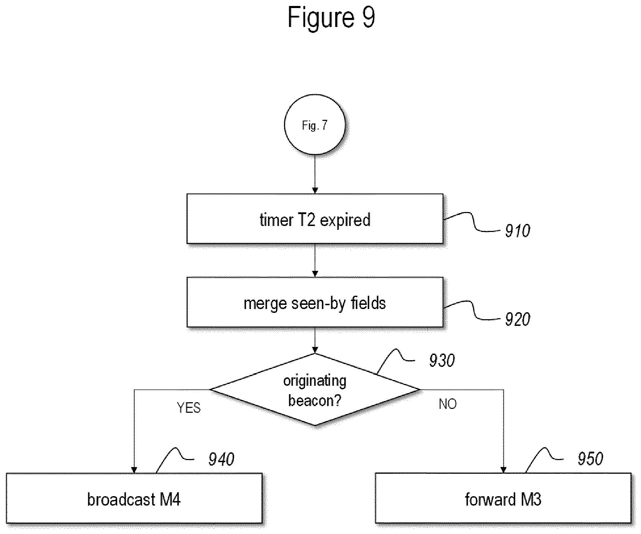

If the message type is found to be "M3", i.e. an upstream ripple message intended to synchronize the "seen by" information, the receiving beacon apparatus will assess **741,** preferably on the basis of a unique identifier of the message or the specific distribution flow, whether the message has been received before. If this is the case, no further action is necessary. If this is not the case, the receiving beacon apparatus starts or restarts **742** a timer T2. The received "M3" message is buffered **743,** and its "Seen by" field is merged (e.g., by OR'ing bitmaps) **744** with the corresponding fields of other "M3" messages that may be received during the time allowed by timer T2 (see also Figure 9).

If the message type is found to be "M4", i.e. a downstream "third ripple" message, the receiving beacon apparatus will assess **751,** preferably on the basis of a unique identifier of the message or the specific distribution flow, whether the message has been received before. If this is the case, the receiving beacon apparatus evaluates **752** the call list, and thenceforth uses this call list to display the relevant information to personnel that checks in at that beacon apparatus. If it is not the case, the "M4" message is retransmitted to further its distribution to the other members of the beacon group.

As illustrated in Figure 8, timer T1 allows any beacon apparatus that has transmitted an M2 message to determine whether it is the last beacon apparatus in a given sector of the network. The timer will only be allowed to expire (without being stopped at step **732** described above), if no copies of the transmitted M2 message are received back from any neighboring beacon apparatus. Upon expiry **810** of the timer, the transmitting beacon checks **820** whether it is the originating beacon apparatus, as indicated by flag F1 (see Figure 5). If this is the case, it activates local mode **830.** If the beacon is not operating in local mode, the failure to receive any copies of its own M2 message is an indication that the ripple has reached the end of a sector, and the beacon apparatus should start an upstream ripple by transmitting **840** an M3 message, which includes the most up-to-date version of the "seen by" field for that sector. In this description, the initial M3 message sent out by the beacon apparatus at the end of a sector is also referred to as an "end point message".

As illustrated in Figure 9, timer T2 allows any beacon that has received an M3 message to wait for additional M3 messages before forwarding the (merged) "seen by" information along the upstream ripple. Upon expiry **910** of the timer, the receiving beacon can merge **920** the "seen by" fields of the various received M3 messages (of course, if no further M3 messages are received, no merging has to take place). The beacon apparatus also checks **930** whether it is the beacon that initiated this particular call list distribution flow. If that is the case, the upstream ripple has come to an end, and the beacon apparatus starts the third ripple by broadcasting **940** an M4 message. If it is not the case, the upstream ripple has to continue, and the beacon apparatus relays **950** the updated "seen by" information under the form of an M3 message.

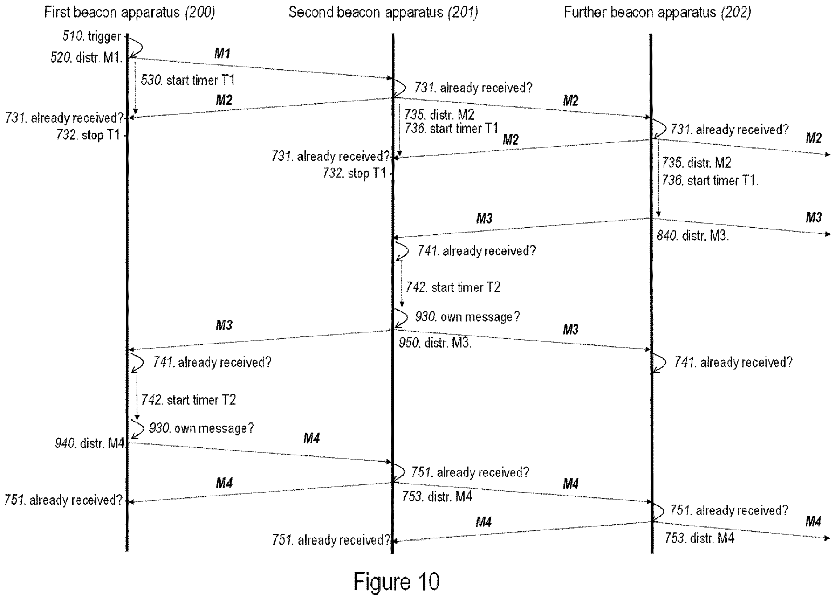

Figure 10 summarizes the previous figures in a single message diagram. The indicated reference numbers refer to the same steps as those appearing in preceding figures, but steps that are not strictly necessary to illustrate the general flow of the protocol have been omitted or simplified for clarity purposes. To further simplify the diagram, the distribution of nurse call information is assumed to start from a first beacon apparatus **200,** and further interactions are limited to a second beacon apparatus **201** and a third beacon apparatus **202.**

Without loss of generality, the interaction is assumed to be triggered by the receipt of a new call **510** at the first beacon apparatus **200.** Upon receiving that trigger, it broadcasts **520** an M1 message and starts **530** timer T1. Timer T1 allows the beacon to detect whether the message it transmits is being redistributed by subsequent beacon apparatus. The M1 message is received by second beacon apparatus **201,** which checks **731** if this particular M1 message (or an M2 counterpart) has been received before, finding that it hasn't. It then broadcasts **735** a corresponding M2 message and starts **736** timer T1. The receipt of this corresponding M2 message by the first beacon apparatus **200** causes the latter to check **731** if this M2 message has been received before and, finding that it is the counterpart of its own M1 message, to stop timer T1. Similarly, the receipt of the M2 message by the third beacon apparatus **202** causes the latter to check **731** if this M2 message has been received before and, finding that it hasn't, broadcast **735** a corresponding M2 message and start **736** its timer T1. The timer T1 of further beacon apparatus **202** will expire before any further echoes of its M2 message are received.

The expiry of timer T1 of the third beacon apparatus **202** starts the upstream part of the message flow. The third beacon apparatus **202** broadcasts **840** an M3 end point message. The receipt of the M3 end point message by the second beacon apparatus **201** (which coincides with the "fourth beacon apparatus" in the description above) causes the latter to check **741** if this M3 message has been received before and, finding that it hasn't, start **742** its timer T2, allowing it to gather further incoming M3 messages (if any) and combine them, before sending out the combined information. Upon expiry of the timer T2, the type of the next broadcast depends **930** on whether the received M3 message(s) corresponded to an M1 message sent at the initiative of the second beacon apparatus **201.** Finding that this is not the case, the second beacon apparatus **201** broadcasts **950** a (combined) M3 message. The receipt of that M3 message by the first beacon apparatus **200** causes the latter to check **741** if this M3 message has been received before and, finding that it hasn't, start **742** its timer T2, allowing it to gather further incoming M3 messages (if any) and combine them, before sending out the combined information. Upon expiry of the timer T2, the type of the next broadcast again depends **930** on whether the received M3 message(s) corresponded to an M1 message sent at the initiative of the first beacon apparatus **200.** Finding that this is the case, the first beacon apparatus **200** broadcasts **940** a (combined) M4 message.

The broadcasting of the M4 message by the first beacon apparatus **200** starts the second downstream part of the message flow. The M4 message is received by second beacon apparatus **201,** which checks 751 if this particular M4 message has been received before, finding that it hasn't. It then broadcasts **753** a corresponding M4 message. The corresponding M4 message is received by further beacon apparatus **202,** which checks **751** if this particular M4 message has been received before, finding that it hasn't. It then again broadcasts **753** a corresponding M4 message. While the first beacon apparatus **200** and the second beacon apparatus **201** also receive the M4 messages sent by their downstream neighbors, they don't act on it, as the check **751** will reveal that they are effectively duplicates.

If wired network links are available and operational between some or all of the relevant beacon apparatus, the protocol can optionally be simplified in some respects. The information merging function ('OR' function) described above with respect to M3 messages, could already be applied to the M2 messages, because many or all of the M2 messages will be received by the initiating beacon, which can therefore easily determine which other beacon apparatus have processed the original M1 message. In fully connected wired networks, where all nodes see each other's M1 and M2 messages, the M3 messages may be skipped for this reason. For the same reason, it is not necessary to relay a message received at a beacon apparatus via a wired interface further via the same wired interface.

While the invention has been described hereinabove with reference to specific embodiments, this is done to illustrate and not to limit the invention, the scope of which is defined by the accompanying claims. The skilled person will readily appreciate that different combinations of features than those described herein are possible without departing from the scope of the claimed invention.

## Claims

1. A system comprising a plurality of beacon apparatus (200, 201, 202) adapted to register nurse calls and a server (300), said plurality of beacon apparatus (200, 201, 202) being connectable to said server (300) by means of a network;
wherein a first beacon apparatus of said plurality of beacon apparatus is configured to:
- detect whether it is operatively connected to said server (300);
- if said first beacon apparatus is operatively connected to said server (300), distribute information pertaining to said nurse calls to said server (300) via said network; and
- otherwise, distribute information pertaining to said nurse calls to at least one target beacon from among said plurality of beacons, which is reachable via said network;
wherein said information pertaining to said nurse calls comprises information pertaining to a call list change detected at said first beacon apparatus, or information pertaining to outstanding calls received at said first beacon apparatus and/or at other beacon apparatus from among said plurality of beacon apparatus; wherein said first beacon apparatus is assigned to a beacon group, and wherein said first beacon apparatus is further configured to select as said at least one target beacon all beacons that are reachable via said network and that are assigned to said beacon group; and
wherein said distributing of information pertaining to said nurse calls to at least one target beacon comprises:
- transmitting (520) information pertaining to said nurse calls (M1) to said at least one target beacon;
- waiting for acknowledgment of receipt (M3) of said information pertaining to said nurse calls from said at least one target beacon; and
- transmitting (940) a confirmation (M4) of said acknowledgment of receipt (M3) to said at least one target beacon.

2. The system according to claim 1, wherein said distributing further comprises verifying if each beacon of said beacon group has received said information pertaining to said nurse calls, and wherein said transmitting of said confirmation is conditional on said verifying.

3. The system according to claim 1 or claim 2, wherein said first beacon apparatus of said plurality of beacon apparatus is further configured to switch to a local mode of operation (830) if no acknowledgment of receipt is received, said local mode of operation causing hardware to provide highly noticeable visual clues indicating that said local mode of operation is active, and, if necessary, use audio-visual signaling to indicate a presence of a call or a need for a personnel intervention.

4. The system according to any of claims 1 to 3, wherein at least two of said plurality of beacon apparatus (200, 201, 202) comprise respective radio frequency transceivers for receiving identification messages from an identification tag, and wherein said network comprises a wireless link provided by said respective radio frequency transceivers.

5. The system according to any of claims 1 to 3, wherein said plurality of beacon apparatus (200, 201, 202) comprise respective radio frequency transceivers for receiving identification messages from an identification tag, and wherein said network consists of wireless links provided by said respective radio frequency transceivers.

6. The system according to any of the preceding claims, wherein said information pertaining to said nurse calls comprises information pertaining to a call list.

7. The system according to any of the preceding claims, wherein said call list change is a newly detected call.

8. The system according to any of the preceding claims, wherein said call list change is a cancellation of a call.

9. The system according to any of the preceding claims, wherein said distributing of information is triggered by a scheduled change in beacon group assignments.

## Patentansprüche

1. System, eine Vielzahl von Bakenvorrichtungen (200, 201, 202) umfassend, die ausgelegt sind, Rufe von Pflegepersonal zu registrieren, und einen Server (300), wobei die Vielzahl von Bakenvorrichtungen (200, 201, 202) mittels eines Netzwerks mit dem Server (300) verbindbar ist;
wobei eine erste Bakenvorrichtung der Vielzahl von Bakenvorrichtungen konfiguriert ist:
- zu erkennen, ob sie operativ mit dem Server (300) verbunden ist;
- wenn die erste Bakenvorrichtung operativ mit dem Server (300) verbunden ist, Verbreiten von Informationen, die sich auf die Rufe von Pflegepersonal beziehen, an den Server (300) über das Netzwerk; und
- ansonsten Verbreiten von Informationen, die sich auf die Rufe von Pflegepersonal beziehen, an mindestens eine Zielbake unter der Vielzahl von Baken, die über das Netzwerk erreichbar sind;
wobei die Informationen, die die Rufe von Pflegepersonal betreffen, sich auf Informationen in Bezug auf eine Ruflistenänderung beziehen, die an einer ersten Bakenvorrichtung erkannt wurde, oder Informationen, die sich auf ausstehende Rufe beziehen, die an der ersten Bakenvorrichtung und/oder an anderen Bakenvorrichtungen unter der Vielzahl von Bakenvorrichtungen empfangen wurden; wobei die erste Bakenvorrichtung einer Bakengruppe zugewiesen ist, und wobei die erste Bakenvorrichtung ferner konfiguriert ist, als die mindestens eine Zielbake alle Baken auszuwählen, die über das Netzwerk erreichbar sind, und die der Bakengruppe zugewiesen sind; und
wobei das Verbreiten der Informationen, die sich auf Rufe des Pflegepersonals beziehen, an mindestens eine Zielbake umfasst:
- Übertragen (520) von Informationen, die sich auf die Rufe von Pflegepersonal beziehen (M1), an die mindestens eine Zielbake;
- Warten auf Empfangsbestätigung (M3) der Informationen, die sich auf die Rufe von Pflegepersonal beziehen, von der mindestens einen Zielbake; und
- Übertragen (940) einer Bestätigung (M4) der Empfangsbestätigung (M3) an die mindestens eine Zielbake.

2. System nach Anspruch 1, wobei das Verbreiten ferner Verifizieren umfasst, ob jede Bake der Bakengruppe die Informationen, die sich auf die Rufe von Pflegepersonal beziehen, empfangen hat, und wobei das Übertragen der Bestätigung von dem Verifizieren abhängig ist.

3. System nach Anspruch 1 oder Anspruch 2, wobei die erste Bakenvorrichtung der Vielzahl von Bakenvorrichtungen ferner konfiguriert ist, in einen lokalen Betriebsmodus (830) zu schalten, wenn keine Empfangsbestätigung empfangen wird, wobei der lokale Betriebsmodus Hardware veranlasst, äußerst auffällige visuelle Hinweise bereitzustellen, die angeben, dass der lokale Betriebsmodus aktiv ist, und, wenn nötig, audiovisuelle Signalgebung verwendet, um ein Vorhandensein eines Rufs oder einer Notwendigkeit einer Intervention durch Personal angibt.

4. System nach einem der Ansprüche 1 bis 3, wobei mindestens zwei der Vielzahl von Bakenvorrichtungen (200, 201, 202) jeweilige Funkfrequenzsendeempfänger zum Empfangen von Identifikationsnachrichten von einem Identifikationstag umfassen, und wobei das Netzwerk eine drahtlose Verbindung umfasst, die von jeweiligen Funkfrequenzsendeempfängern bereitgestellt wird.

5. System nach einem der Ansprüche 1 bis 3, wobei die Vielzahl von Bakenvorrichtungen (200, 201, 202) jeweilige Funkfrequenzsendeempfänger zum Empfangen von Identifikationsnachrichten von einem Identifikationstag umfassen, und wobei das Netzwerk aus drahtlosen Verbindungen besteht, die von den jeweiligen Funkfrequenzsendeempfängern bereitgestellt werden.

6. System nach einem der vorstehenden Ansprüche, wobei die Informationen, die sich auf die Rufe von Pflegepersonal beziehen, Informationen umfassen, die sich auf eine Rufliste beziehen.

7. System nach einem der vorstehenden Ansprüche, wobei die Ruflistenänderung ein neu erkannter Ruf ist.

8. System nach einem der vorstehenden Ansprüche, wobei die Ruflistenänderung eine Stornierung eines Rufs ist.

9. System nach einem der vorstehenden Ansprüche, wobei das Verbreiten von Informationen von einer planmäßigen Änderung der Bakengruppenzuweisungen ausgelöst wird.

## Revendications

1. Système comprenant une pluralité d'appareils formant balises (200, 201, 202) conçus pour enregistrer des appels d'infirmière et un serveur (300), ladite pluralité d'appareils formant balises (200, 201, 202) pouvant être reliée audit serveur (300) au moyen d'un réseau ;
dans lequel un premier appareil formant balise de ladite pluralité d'appareils formant balises est configuré pour :
- détecter s'il est relié de manière opérationnelle audit serveur (300) ;
- si ledit premier appareil formant balise est relié de manière opérationnelle audit serveur (300), distribuer des informations se rapportant auxdits appels d'infirmière audit serveur (300) via ledit réseau ; et
- autrement, distribuer des informations se rapportant auxdits appels d'infirmière à au moins une balise cible parmi ladite pluralité de balises, qui est accessible via ledit réseau ;
dans lequel lesdites informations se rapportant auxdits appels d'infirmière comprennent des informations se rapportant à un changement de liste d'appels détecté au niveau dudit premier appareil formant balise, ou des informations se rapportant à des appels en suspens reçus au niveau dudit premier appareil formant balise et/ou au niveau d'un autre appareil formant balise parmi ladite pluralité d'appareils formant balises ; dans lequel ledit premier appareil formant balise est attribué à un groupe de balises, et dans lequel ledit premier appareil formant balise est en outre configuré pour sélectionner en tant que dite au moins une balise cible toutes les balises qui sont accessibles via ledit réseau et qui sont attribuées audit groupe de balises ; et
dans lequel ladite distribution d'informations se rapportant auxdits appels d'infirmière à au moins une balise cible comprend :
- l'émission (520) d'informations se rapportant auxdits appels d'infirmière (M1) vers ladite au moins une balise cible ;
- l'attente d'un accusé de réception (M3) desdites informations se rapportant auxdits appels d'infirmière en provenance de ladite au moins une balise cible ; et
- l'émission (940) d'une confirmation (M4) dudit accusé de réception (M3) vers ladite au moins une balise cible.

2. Système selon la revendication 1, dans lequel ladite distribution comprend en outre la vérification si chaque balise dudit groupe de balises a reçu lesdites informations se rapportant auxdits appels d'infirmière, et dans lequel ladite émission de ladite confirmation est conditionnée à ladite vérification.

3. Système selon la revendication 1 ou la revendication 2, dans lequel ledit premier appareil formant balise de ladite pluralité d'appareils formant balises est en outre configuré pour commuter sur un mode de fonctionnement local (830) si aucun accusé de réception de reçu n'est reçu, ledit mode de fonctionnement local amenant du matériel à fournir des indices visuels fortement perceptibles indiquant que ledit mode de fonctionnement local est actif et, si nécessaire, à utiliser une signalisation audiovisuelle pour indiquer une présence d'un appel ou un besoin d'une intervention de personnel.

4. Système selon l'une quelconque des revendications 1 à 3, dans lequel au moins deux de ladite pluralité d'appareils formant balises (200, 201, 202) comprennent des émetteurs-récepteurs radiofréquence respectifs pour recevoir des messages d'identification en provenance d'une étiquette d'identification, et dans lequel ledit réseau comprend une liaison sans fil fournie par lesdits émetteurs-récepteurs radiofréquence respectifs.

5. Système selon l'une quelconque des revendications 1 à 3, dans lequel ladite pluralité d'appareils formant balises (200, 201, 202) comprend des émetteurs-récepteurs radiofréquence respectifs pour recevoir des messages d'identification en provenance d'une étiquette d'identification, et dans lequel ledit réseau consiste en liaisons sans fil fournies par lesdits émetteurs-récepteurs radiofréquence respectifs.

6. Système selon l'une quelconque des revendications précédentes, dans lequel lesdites informations se rapportant auxdits appels d'infirmière comprennent des informations se rapportant à une liste d'appels.

7. Système selon l'une quelconque des revendications précédentes, dans lequel ledit changement de liste d'appels est un appel nouvellement détecté.

8. Système selon l'une quelconque des revendications précédentes, dans lequel ledit changement de liste d'appels est une annulation d'un appel.

9. Système selon l'une quelconque des revendications précédentes, dans lequel ladite distribution d'informations est déclenchée par un changement planifié d'attributions de groupe de balises.
